# EUROPEAN PATENT APPLICATION

(11) **EP 4 484 405 A1**
(43) Date of publication of application: **01.01.2025**
(21) Application number: 23760355.0
(22) Date of filing: 21.02.2023
(51) Int. Cl.: C07C 67/56, C07C 67/54, C07C 69/86

(54) **METHOD FOR PREPARING RECYCLED BIS(2-HYDROXYETHYL) TEREPHTHALATE THROUGH MULTI-STEP DEPOLYMERIZATION**

(30) Priority: 25.02.2022 KR 20220025351
(71) Applicant: SK Chemicals Co., Ltd., Seongnam-si, Gyeonggi-do 13494 (KR)
(72) Inventor: KIM, Ji-Hun, Seongnam-si, Gyeonggi-do 13494 (KR); PARK, Kwang-Woo, Seongnam-si, Gyeonggi-do 13494 (KR); LEE, Joong Ki, Seongnam-si, Gyeonggi-do 13494 (KR); JIN, Yuntae, Seongnam-si, Gyeonggi-do 13494 (KR)
(74) Representative: Dehns
(86) International application number: PCT/KR2023/002449
(87) International publication number: WO 2023/163481

(57) **Abstract**

A method, according to one embodiment, depolymerizes waste polyester by means of a multi-step reaction, wherein a latter reaction is carried out at a low temperature, and ion exchange, etc., is further carried out, thereby enabling the provision of high-purity and high-quality recycled bis(2-hydroxyethyl) terephthalate having reduced generation of diethylene glycol-derived impurities and oligomers.

## Description

### Technical Field

The present invention relates to a process for preparing recycled bis(2-hydroxyethyl) terephthalate (BHET) with high purity and high quality using waste polyester.

### Background Art

Polyester, among polymers commonly used in modern life, is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world are preparing regulations and plans for recycling waste plastic resources, including waste polyester. Although physical or chemical methods are used as methods of recycling waste polyester, physical recycling methods cannot guarantee purity and are not widely used.

In chemical recycling methods, the ester bond of waste polyester is broken to depolymerize it. Reactions such as glycolysis, hydrolysis, methanolysis, and aminolysis are used. Glycolysis among them is to decompose waste polyester by adding a glycol such as ethylene glycol or diethylene glycol at high temperatures. A reaction product containing mainly bis(2-hydroxyethyl) terephthalate (BHET) is obtained.

The bis(2-hydroxyethyl) terephthalate may be used as a raw material for preparing unsaturated polyester or ester polyol after the crystallization or purification thereof.

### [Prior Art Document]

(Patent Document 1) Korean Patent No. 1386683
(Patent Document 2) U.S. Patent No. 7211193
(Non-patent Document 1) Park, S.H., Kim, S.H., Polyethylene terephthalate) recycling for high value added textiles, Fashion and Textiles 1, 1 (2014)

### Disclosure of Invention

### Technical Problem

In general, a product of the depolymerization reaction of a waste polyester resin comprises a significant amount of oligomers such as dimers and trimers in addition to bis(2-hydroxyethyl) terephthalate (BHET). Since diethylene glycol (DEG) is formed at a high depolymerization temperature, it is inevitable that side-reaction products derived from diethylene glycol are formed.

For example, a diethylene glycol ester (DEG ester) causes a decrease in physical properties in polymerizing a regenerated polyester resin, the extent of which is greater in the case of polyester copolymers. In addition, it makes the color of the polymerization raw materials dark and yellow; thus, the use thereof is inevitably limited.

As a result of research conducted by the present inventors to solve this problem, it has been discovered that BHET can be produced with high purity by carrying out the depolymerization reaction in multiple stages, while significantly lowering the temperature of the latter stage, thereby reducing the formation of diethylene glycol and impurities derived therefrom.

In addition, the present inventors have been able to prepare BHET with enhanced quality in terms of color by further carrying out ion exchange and distillation of unreacted glycol after the depolymerization reaction, thereby reducing the formation of oligomers and removing chromophores.

Accordingly, an object of the present invention is to provide a process for preparing recycled bis(2-hydroxyethyl) terephthalate (BHET) with high purity and high quality using waste polyester through a depolymerization reaction in multiple stages, along with separation and purification steps.

### Solution to Problem

The present invention provides a process for preparing recycled bis(2-hydroxyethyl) terephthalate, which comprises (1) subjecting waste polyester to depolymerization by a first glycolysis reaction at a temperature of 180°C to 200°C to obtain a first reactant; (2) subjecting the first reactant to depolymerization by a second glycolysis reaction at a temperature of 150°C to 170°C to obtain a second reactant; (3) subjecting the second reactant to ion exchange through an ion-exchange resin to obtain a third reactant; (4) removing an unreacted glycol from the third reactant through distillation at a temperature of 150°C or lower to obtain a fourth reactant; and (5) subjecting the fourth reactant to distillation to obtain crude bis(2-hydroxyethyl) terephthalate.

In addition, the present invention provides recycled bis(2-hydroxyethyl) terephthalate, which is prepared by the above process, wherein the peak area fraction of bis(2-hydroxyethyl) terephthalate is 96% or more, and the peak area fraction of diethylene glycol ester compounds is less than 2% in total, when measured by high-performance liquid chromatography (HPLC).

### Advantageous Effects of Invention

According to the process of the present invention, recycled bis(2-hydroxyethyl) terephthalate (BHET) can be produced with high purity by carrying out the depolymerization reaction in multiple stages, while significantly lowering the temperature of the latter stage, thereby reducing the formation of diethylene glycol and impurities derived therefrom. In addition, according to the process of the present invention, it is possible to prepare bis(2-hydroxyethyl) terephthalate (BHET) with enhanced quality in terms of color by further carrying out ion exchange and distillation of an unreacted glycol after the depolymerization reaction, thereby reducing the formation of oligomers and removing chromophores.

Accordingly, not only can the present invention depolymerize waste polyester for recycling resources, but also polyester resins and products of excellent quality are produced using the recycled bis(2-hydroxyethyl) terephthalate produced according to the present invention as a raw material.

### Brief Description of Drawings

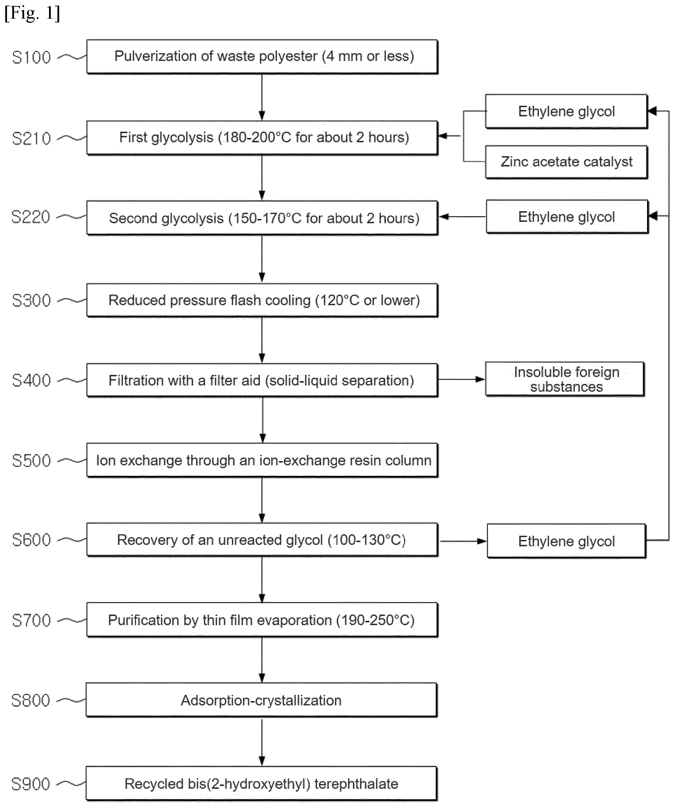

Fig. 1 illustrates the process for preparing recycled bis(2-hydroxyethyl) terephthalate according to an embodiment.

### Best Mode for Carrying out the Invention

Hereinafter, various embodiments and examples will be described in detail by referring to the drawings.

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element and/or component, unless specifically stated to the contrary.

The process for preparing recycled bis(2-hydroxyethyl) terephthalate according to the present invention comprises (1) subjecting waste polyester to depolymerization by a first glycolysis reaction at a temperature of 180°C to 200°C to obtain a first reactant; (2) subjecting the first reactant to depolymerization by a second glycolysis reaction at a temperature of 150°C to 170°C to obtain a second reactant; (3) subjecting the second reactant to ion exchange through an ion-exchange resin to obtain a third reactant; (4) removing an unreacted glycol from the third reactant through distillation at a temperature of 150°C or lower to obtain a fourth reactant; and (5) subjecting the fourth reactant to distillation to obtain crude bis(2-hydroxyethyl) terephthalate.

Prior to step (1), a step of pulverizing the waste polyester to a size of a certain level or below may be further carried out. In addition, a step of cooling the second reactant obtained in step (2) to a certain temperature or lower may be further carried out. In addition, prior to step (3), a step of removing insoluble foreign substances from the second reactant through filtration may be further carried out. In addition, after the distillation in step (5), a step of adsorbing-crystallizing the crude bis(2-hydroxyethyl) terephthalate may be further carried out.

Fig. 1 illustrates the process for preparing recycled bis(2-hydroxyethyl) terephthalate according to an embodiment. Referring to Fig. 1, first, waste polyester is pulverized to a size of 4 mm or less to be prepared (S 100), ethylene glycol is added thereto, which is then subjected to a first glycolysis reaction at a temperature of 180°C to 200°C in the presence of a zinc acetate catalyst for about 2 hours (S210), and ethylene glycol is further added thereto, which is then subjected to a second glycolysis reaction at a temperature of 150°C to 170°C for about 2 hours (S220). Thereafter, it is cooled to 120°C or lower using a reduced pressure flash (S300), a small amount of a filter aid is added thereto, which is then filtered to separate insoluble foreign substances through solid-liquid separation (S400), and it is passed through a column filled with ion-exchange resin to carry out ion exchange (S500). Then, an unreacted glycol is recovered at a temperature of 100°C to 130°C (S600), purification is carried out by thin film distillation at 190°C to 250°C (S700), and, finally, an adsorption-crystallization step is carried out (S800) to obtain bis(2-hydroxyethyl) terephthalate with high purity and high quality.

Hereinafter, each step will be described in detail.

### Preparation of waste polyester

Waste polyester used as a raw material in the present invention may be obtained from a polyester material product discarded after use.

For example, the waste polyester may be obtained from products such as beverage bottles, fabrics, films, cases, boxes, partitions, shelves, protective panels, packaging materials, building materials, and interior and exterior materials made of various polyester materials discarded after having been used by consumers.

The waste polyester material may be pretreated before being subjected to a depolymerization step.

First, once other plastics, metals, and foreign substances mixed in the waste polyester material have been removed, it is washed and screened according to detailed characteristics such as color, if necessary.

The waste polyester material thus screened is put into a pulverizer to be pulverized into small flakes. The flakes of waste polyester thus obtained may be screened to a desired particle size or less using a mesh. The size of the mesh may be, for example, 4 mm or less, 3 mm or less, or 2 mm or less.

The screened flakes may be washed, dried by hot air or the like, and then put into a depolymerization step.

Meanwhile, the waste polyester subjected to a glycolysis reaction may have a controlled particle size. For example, the waste polyester may be pulverized by the pretreatment step as described above to have a flake shape.

Specifically, the particle diameter of the waste polyester may be 4 mm or less, 3 mm or less, 2 mm or less, or 1 mm or less. Within the above particle diameter range, a glycolysis reaction under relatively low-temperature conditions is possible. For example, the temperature condition of the first glycolysis reaction may be adjusted to 195°C or lower, 190°C or lower, 185°C or lower, or 180°C, and the temperature condition of the subsequent second glycolysis reaction may be adjusted to 160°C or lower or 150°C or lower. In addition, within the above particle diameter range, a glycolysis reaction within a relatively short period of time is possible. For example, the period of time for the first and second glycolysis reactions may be 3 hours or less, 2 hours or less, or 1 hour or less, from the point when the appropriate temperature is reached.

In addition, the waste polyester may have a fine structure like a fiber. For example, the waste polyester may be a waste fiber or a fibrous material such as a waste banner.

As a specific example, the waste polyester may have a particulate or fibrous form with a particle diameter of 4 mm or less.

The fiber may comprise at least one of a monofilament yarn and a multifilament yarn. The diameter of the monofilament yarn may be, for example, 0.05 denier to 100 denier and may correspond to approximately 0.001 mm to 0.1 mm. Specifically, the monofilament yarn may have a diameter of 0.05 denier to 7 denier or 7 denier to 100 denier. The diameter of the multifilament yarn may be, for example, 1 denier to 10,000 denier and may correspond to approximately 0.01 mm to 1 mm. Specifically, the multifilament yarn may have a diameter of 0.01 denier to 0.2 denier or 0.2 denier to 1 denier.

If the depolymerization is carried out as the particle diameter or diameter of waste polyester is adjusted within the specific range, solvation can be expedited even under the conditions of relatively low temperatures and short reaction time.

In particular, according to the subject invention, a two-stage glycolysis reaction (i.e., a first glycolysis reaction and a second glycolysis reaction) is carried out. If the solvation is expedited in the first glycolysis reaction, the transesterification reaction of the waste polyester can be performed under the conditions of lower temperatures and short reaction time in the second glycolysis reaction. Thus, it is possible to significantly reduce the concentration of diethylene glycol (DEG) naturally formed at a common glycolysis reaction temperature and to significantly reduce the content of diethylene glycol ester compounds (DEG esters) in the recycled bis(2-hydroxyethyl) terephthalate finally prepared.

The diethylene glycol ester compounds present in the recycled bis(2-hydroxyethyl) terephthalate acts as a factor that breaks the regularity of a final polymer during the subsequent polymerization of a polyester and a polyester copolymer, thereby deteriorating the thermal resistant characteristics of the final polymer such as melting point (Tm), glass transition temperature (Tg), and the like.

However, polyester resins and products manufactured using the recycled bis(2-hydroxyethyl) terephthalate obtained according to the depolymerization process of the present invention can be polymerized into polymers without unnecessary structural defects as in the case where a virgin, non-recycled raw material is used.

### Depolymerization

According to the process of the present invention, as waste polyester is subjected to the pretreatment of pulverization and to a depolymerization reaction in multiple stages at low temperatures, it is possible to significantly reduce the content of glycol dimers (diethylene glycol) formed during the depolymerization reaction, so that there is an advantage in that the purity of the recycled bis(2-hydroxyethyl) terephthalate finally obtained is increased and side-reaction structures in a subsequent repolymerization into polyester are minimized.

According to an embodiment, the depolymerization comprises subjecting waste polyester to depolymerization through a first glycolysis reaction at a high temperature (180 to 200°C) to obtain a first reactant; and subjecting the first reactant to depolymerization at a low temperature (150 to 170°C) through a second glycolysis reaction to obtain a second reactant.

As is well known, the glycolysis reaction refers to a chemical reaction in which a polymer chain or the like is decomposed by a glycol. The glycol may comprise, for example, at least one selected from the group consisting of ethylene glycol, propylene glycol, and diethylene glycol.

A catalyst may be used in the glycolysis reaction. The catalyst may be a metal catalyst, for example, a metal salt catalyst or a metallic organic catalyst. Specifically, the catalyst may be an acetate, carbonate, oxide, or hydroxide of a metal, and the metal may be an alkali metal, an alkaline earth metal, or a transition metal.

As a specific example, the catalyst comprises a metal acetate or an anhydride or a hydride thereof. More specifically, it may be at least one selected from the group consisting of zinc acetate, sodium acetate, cobalt acetate, and manganese acetate, or in the form of a hydrate or anhydride thereof.

The total weight of the glycol added may be 1, 2, or 3 times the weight of the waste polyester resin or more and may be 7, 5, or 4 times or less. For example, the weight of the glycol added may be 1 to 7 times, specifically, 2 to 5 times, more specifically, 3 to 4 times, relative to the weight of the waste polyester resin.

In addition, the weight of the catalyst added may be 0.01 part by weight or more, 0.1 part by weight or more, 0.2 part by weight or more, or 0.3 part by weight or more, and may be 5 parts by weight or less, 1 part by weight or less, 0.7 part by weight or less, 0.5 part by weight or less, or 0.4 part by weight or less, relative to 100 parts by weight of the waste polyester resin. For example, the weight of the catalyst added may be 0.1 part by weight to 1 part by weight, specifically, 0.2 part by weight to 0.7 part by weight, relative to 100 parts by weight of the waste polyester resin. More specifically, the catalyst may be used in an amount of 0.2 part by weight to 0.4 part by weight relative to 100 parts by weight of the waste polyester.

The temperature during the first glycolysis reaction may be 170°C or higher, 180°C or higher, or 190°C or higher, and may be 205°C or lower, 200°C or lower, 195°C or lower, or 190°C or lower. For example, the temperature during the first glycolysis reaction may be 180°C to 200°C, specifically, 180°C to 195°C, more specifically, 180°C to 190°C.

In addition, the temperature during the second glycolysis reaction may be 140°C or higher, 150°C or higher, or 160°C or higher, and may be 170°C or lower or 160°C or lower. For example, the temperature during the second glycolysis reaction may be 150°C to 170°C, specifically, 150°C to 160°C, more specifically, 150°C to 155°C.

The period of time required for the first and second glycolysis reactions may be 1 hour or more or 2 hours or more, and may be 4 hours or less or 3 hours or less, from the point when the appropriate temperature is reached. For example, the period of time required for the first and second glycolysis reactions may be 1 hour to 4 hours, specifically, 1 hour to 3 hours, more specifically, 1 hour to 2 hours, from the point when the appropriate temperature is reached.

As a specific example, the first glycolysis reaction may be carried out at a temperature of 180°C to 190°C for 1 hour to 3 hours. In addition, the second glycolysis reaction may be carried out at a temperature of 150°C to 160°C for 1 hour to 3 hours.

As an example, the first glycolysis reaction may be carried out in the presence of a zinc acetate anhydride catalyst. As a specific example, the first glycolysis reaction may be carried out at a temperature of 180°C to 200°C for 1 hour to 3 hours in the presence of a zinc acetate anhydride catalyst. The zinc acetate anhydride may be used in an amount of 0.2 part by weight to 0.4 part by weight relative to 100 parts by weight of the waste polyester. In addition, the second glycolysis reaction may be carried out at a temperature of 140°C to 160°C for 1 hour to 3 hours upon the further addition of ethylene glycol without an additional catalyst.

### Cooling and filtration

The second reactant obtained through the depolymerization may then be cooled and used in the next step.

The cooling temperature may be, for example, 150°C or lower, 140°C or lower, 130°C or lower, 120°C or lower, 110°C or lower, 100°C or lower, and may be 50°C or higher, 60°C or higher, 70°C or higher, 80°C or higher, or 90°C or higher.

As an example, the cooling may be carried out through a reduced pressure flash process. Specifically, the temperature of the second reactant may be lowered by evaporating ethylene glycol by applying a vacuum through the reduced pressure flash process.

For example, the second reactant may be further subjected to a step of cooling to 120°C or lower through reduced pressure flash prior to subsequent steps. More specifically, the temperature of the second reactant may be lowered to 110°C or lower or 100°C or lower by the reduced pressure flash process.

The pressure condition of the reduced pressure flash process may be, for example, 200 Torr or less, 100 Torr or less, or 50 Torr or less, specifically, 10 Torr to 200 Torr, 10 Torr to 100 Torr, or 10 Torr to 50 Torr.

Thereafter, insoluble foreign substances may be removed from the cooled second reactant through filtration. As a specific example, prior to the ion exchange in step (3), a step of cooling the second reactant to 120°C or lower and filtering it upon the addition of a filter aid may be further carried out. As a result, fine particles and insoluble organic substances present in the second reactant can be filtered out by solid-liquid separation.

Known ingredients such as diatomaceous earth, perlite, and asbestos powder may be used as the filter aid. For example, 0.1 part by weight to 2.0 parts by weight of the filter aid may be added to 100 parts by weight of the second reactant.

Since bis(2-hydroxyethyl) terephthalate (BHET) or oligomers obtained through the depolymerization reaction is present in a solid form at room temperature, it is difficult to separate foreign substances at room temperature. Thus, it is preferable to separate them at a temperature condition of 90°C to 150°C, more specifically, 110°C to 150°C. In addition, if the above temperature range is maintained, the removal of insoluble foreign substances may be facilitated thanks to good flowability.

Various methods and devices may be used in the removal of insoluble foreign substances through solid-liquid separation. For example, a device such as a pressurized filter, a centrifugal separator, a filter press, a belt press, or the like may be used. But it is not limited thereto as long as any method capable of separating foreign substances is used.

### Ion exchange

The second reactant, which has been depolymerized, cooled, and filtered, is subjected to ion exchange through an ion-exchange resin to obtain a third reactant.

As it is subjected to the ion exchange, ionic impurities present in the second reactant, specifically, catalysts and foreign substances, may be removed.

As is well known, an ion-exchange resin refers to a resin or polymer that serves as a medium for ion exchange. The ion-exchange resin may comprise a cation-exchange resin, an anion-exchange resin, an amphoteric ion-exchange resin, a chelate resin, or the like.

The cation-exchange resin may comprise a strongly acidic cation-exchange resin having a sulfonic acid group (-SO₃H) and a weakly acidic cation-exchange resin having a carboxyl group (-COOH). The anion-exchange resin may comprise a strongly basic anion-exchange resin in the form of a quaternary ammonium salt and a weakly basic anion-exchange resin having a primary to tertiary amino group.

As a specific example, the ion-exchange resin may comprise at least one selected from the group consisting of a strongly acidic cation-exchange resin, a weakly acidic cation-exchange resin, and a chelate resin.

According to an embodiment, the ion exchange is carried out by adding an ion-exchange resin to the second reactant.

The weight of the ion-exchange resin added may be 1, 3, or 5 times the weight of the catalyst added in the depolymerization reaction or more, and may be 20, 15, 10, or 8 times or less. For example, the weight of the ion-exchange resin added may be 1 to 20 times, specifically, 3 to 15 times, more specifically, 5 to 8 times, relative to the weight of the catalyst added in the depolymerization reaction.

In addition, the weight of the ion-exchange resin added may be 1 part by weight or more, 3 parts by weight or more, or 5 parts by weight or more, and may be 50 parts by weight or less, 20 parts by weight or less, 15 parts by weight or less, 10 parts by weight or less, or 7 parts by weight or less, relative to 100 parts by weight of the waste polyester resin employed in the depolymerization reaction.

As a specific example, the ion-exchange resin may be used in an amount of 1 part by weight to 20 parts by weight relative to 100 parts by weight of the waste polyester.

According to another embodiment, the ion exchange is carried out by using a column containing an ion-exchange resin.

Specifically, the column may be filled with particles of an ion-exchange resin, and ion exchange may be carried out while the second reactant passes through the column.

The particle diameter of the ion-exchange resin particles may be, for example, 0.3 mm to 1.5 mm, more specifically, 0.6 mm to 0.9 mm.

The temperature for ion exchange may be, for example, 140°C or lower, 130°C or lower, 120°C or lower, 110°C or lower, 100°C or lower, and may be 50°C or higher, 60°C or higher, 70°C or higher, 80°C or higher, or 90°C or higher.

### Removal of unreacted glycol

An unreacted glycol is removed from the third reactant through distillation to obtain a fourth reactant.

Since an unreacted glycol still remains in the depolymerization resultant after filtration thereof in the previous step, it is necessary to remove the same from the reactant prior to the next step.

In addition, it is necessary to perform a step of recovering an unreacted glycol for an economical depolymerization process. That is, it is possible to recover and reuse a glycol, among glycols such as ethylene glycol, propylene glycol, diethylene glycol, or the like previously employed in the depolymerization, that remains without participating in the glycolysis reaction.

The distillation to remove the unreacted glycol may be carried out by, for example, vacuum distillation. A glass distillation apparatus or a rotary evaporator may be used for this purpose.

As the vacuum distillation to remove the unreacted glycol is carried out at a temperature of 150°C or lower, the purity of BHET can be enhanced by further reducing the formation of diethylene glycol and impurities derived therefrom. For example, the vacuum distillation to remove the unreacted glycol may be carried out at a temperature of 150°C lower, 130°C lower, or 120°C lower, and 80°C higher, 90°C higher, 100°C higher, or 110°C higher. Specifically, the temperature during the distillation to remove the unreacted glycol may be 80°C to 190°C or 90°C to 150°C. As a more specific example, the distillation to remove the unreacted glycol may be carried out at a temperature of 100°C to 130°C.

The pressure during the distillation to remove the unreacted glycol may be, for example, 0.1 Torr to 200 Torr, more specifically, 0.5 Torr to 30 Torr.

### Distillation

The fourth reactant from which the unreacted glycol has been removed is subjected to distillation to obtain crude bis(2-hydroxyethyl) terephthalate.

Various methods may be used for the distillation, while a distillation method to make a mixture to be separated into a thin film for increasing its surface area in contact with a heat source may be used.

For example, the distillation may be carried out by thin film evaporation, falling film evaporation, or short path evaporation. For this purpose, a thin film evaporator, a falling film evaporator, and a short path evaporator may be used, respectively.

As a specific example, the distillation to obtain bis(2-hydroxyethyl) terephthalate may be carried out by thin film evaporation. Specifically, a mixture fed to the evaporator of the thin film evaporator forms a thin film on the inner wall of the thin film evaporator by the wiper rotor. Then, distillation is carried out under appropriate temperature conditions by heating. In addition, a condenser for recovering the evaporated material may be provided inside the thin film evaporator.

The thin film evaporation may be carried out by short path evaporation. Since such a short path and thin film evaporation has a short residence time and enables vacuum distillation using a high vacuum, it is possible to separate high-boiling or high-molecular-weight materials that are hardly separated by other distillation methods while minimizing the change of the reactants by heat. In addition, if the pressure inside a thin film evaporator is lowered, there is an advantage in that the vapor pressure of a material is reduced, which allows evaporation to take place at a lower temperature than its original boiling point.

As a specific example, the fourth reactant is fed to a short path and thin film evaporator, and a wiper for forming a thin film is rotated at 300 rpm or more. As a result, a vaporized material and a non-vaporized material can be separated from each other.

The internal thin film temperature of the upper thin film evaporation apparatus during the thin film evaporation may be, for example, 100°C or higher, 110°C or higher, 120°C or higher, or 125°C or higher, and may be 250°C or lower, 200°C or lower, 150°C or lower, or 135°C or lower, specifically, 150°C to 250°C, 190°C to 250°C, or 180°C to 220°C.

In addition, the internal pressure of the upper thin film evaporation apparatus during the thin film evaporation may be, for example, 0.005 Torr to 5.0 Torr, specifically, 0.05 Torr to 5.0 Torr, 0.05 Torr to 1.5 Torr, or 0.05 Torr to 1 Torr. More specifically, the distillation to obtain crude bis(2-hydroxyethyl) terephthalate may be carried out by thin film evaporation under a pressure of 0.05 Torr to 0.4 Torr.

### Adsorption-crystallization

The crude bis(2-hydroxyethyl) terephthalate is subjected to an adsorption-crystallization step to be provided as recycled bis(2-hydroxyethyl) terephthalate with high purity and high quality.

For example, the adsorption-crystallization may be carried out by adding an adsorbent using water as a solvent, filtering, and crystallization.

Various solvents may be used for the adsorption-crystallization, but a solvent capable of dissolving bis(2-hydroxyethyl) terephthalate is preferably used as a solvent. As a specific example, in order to obtain the final reactant, water as a solvent is added to the crude bis(2-hydroxyethyl) terephthalate, which is dissolved by heating, and an adsorbent is added thereto, followed by subjecting the solution obtained by filtration to cooling-crystallization and final filtration. As a result, recycled bis(2-hydroxyethyl) terephthalate with high purity can be obtained.

Water may be added in an amount of 100 parts by weight to 500 parts by weight, specifically, 200 parts by weight to 400 parts by weight, more specifically, 300 parts by weight to 350 parts by weight, relative to 100 parts by weight of the crude bis(2-hydroxyethyl) terephthalate.

In addition, the dissolution temperature may be 50°C to 95°C, specifically, 60°C to 85°C, more specifically, 70°C to 75°C.

The adsorbent added may serve to adsorb and remove other foreign substances. It may be added in an amount of 0.1 part by weight to 3 parts by weight relative to 100 parts by weight of the crude bis(2-hydroxyethyl) terephthalate. The type and form of the adsorbent are not particularly limited. For example, activated carbon may be used.

### Recycled bis(2-hydroxyethyl) terephthalate

Bis(2-hydroxyethyl) terephthalate (BHET) obtained by the depolymerization of waste polyester is referred to as recycled bis(2-hydroxyethyl) terephthalate (recycled BHET, or r-BHET), which needs to be understood as distinct from a pure BHET compound.

Specifically, recycled BHET may contain reagents or solvents used in various chemical steps during the depolymerization of waste polyester, or by-products formed by side reactions with them. These impurities may remain in trace amounts even after several rounds of purification. Thus, recycled BHET generally contains trace amounts of organic and inorganic impurities in addition to BHET as the main component. For this reason, recycled BHET can also be viewed as a kind of "composition" comprising two or more components, i.e., a "BHET composition."

Specifically, recycled BHET may comprise trace amounts of a heterogeneous organic component, such as BHET analogs such as monohydroxyethyl terephthalic acid (MHET), BHET dimers, BHET trimers, by-products such as diethylene glycol esters, metal ions as inorganic components, and residual solvent components in addition to BHET as the main component.

However, the recycled BHET finally obtained according to the present invention has high purity, comprising organic impurities, especially diethylene glycol and by-products derived therefrom (DEG esters or the like) or other oligomers and inorganic substances at a certain level or less, and it has excellent color quality.

This can be confirmed by analyzing the final product obtained from the above process with high-performance liquid chromatography (HPLC) and calculating the relative ratio between peak areas for the respective components. Specifically, the content of each component can be derived by measuring the fraction (%) of a peak area out of the total peak area in a spectrum obtained using high-performance liquid chromatography.

For example, the recycled bis(2-hydroxyethyl) terephthalate produced by the above process may have a peak area fraction of bis(2-hydroxyethyl) terephthalate of 90% or more, 93% or more, or 95% or more when measured by HPLC.

According to an embodiment, the recycled bis(2-hydroxyethyl) terephthalate produced by the above process may have a peak area fraction of bis(2-hydroxyethyl) terephthalate of 96% or more when measured by HPLC. Specifically, the peak area fraction of bis(2-hydroxyethyl) terephthalate may be 96.5% or more, 97% or more, 97.5% or more, or 98% or more.

In addition, the recycled bis(2-hydroxyethyl) terephthalate produced by the above process may have a peak area fraction of organic impurities measured by HPLC of less than 5% in total, specifically, less than 4%, less than 3%, less than 2%, less than 1%, or less than 0.7%, in total.

According to an embodiment, the recycled bis(2-hydroxyethyl) terephthalate produced by the above process may have a peak area fraction of diethylene glycol ester compounds of less than 2% in total when measured by HPLC. Specifically, the peak area fraction of diethylene glycol ester compounds may be less than 1.5%, less than 1%, less than 0.8%, or less than 0.7%, in total.

The diethylene glycol ester compounds may be, for example, a condensate between an aromatic dicarboxylic acid such as terephthalic acid and diethylene glycol. As another example, the diethylene glycol ester compounds may be a condensate between an aromatic dicarboxylic acid such as terephthalic acid and a glycol (e.g., ethylene glycol) in addition to diethylene glycol. As a specific example, the diethylene glycol ester compounds may comprise at least one of 2-hydroxyethyl[2-(2-hydroxyethoxy)ethyl] terephthalate and bis[2-(2-hydroxyethoxy)ethyl]benzene-1,4-dicarboxylate.

In addition, the recycled bis(2-hydroxyethyl) terephthalate produced by the above process may have a peak area fraction of oligomers of 3% or less in total when measured by HPLC. This is a remarkably superior level in terms of purity and quality as compared with the oligomeric materials present in a product obtained by depolymerization in the conventional methods ranging from 10 to 20%.

Specifically, the recycled bis(2-hydroxyethyl) terephthalate produced by the above process may have a peak area fraction of BHET dimers of less than 3%, less than 2%, less than 1%, or less than 0.7%, when measured by HPLC. In addition, the recycled bis(2-hydroxyethyl) terephthalate produced by the above process may have a peak area fraction of BHET trimers of less than 1%, less than 0.5%, less than 0.3%, less than 0.1%, or 0%, when measured by HPLC.

In addition, the final product may further comprise impurities having a structure similar to that of bis(2-hydroxyethyl) terephthalate. For example, it may comprise at least one selected from the group consisting of monohydroxyethyl terephthalic acid (MHET), bis(2-hydroxypropyl) terephthalate, and monohydroxyethylethoxy terephthalic acid. The impurities having a structure similar to that of bis(2-hydroxyethyl) terephthalate may have a peak area fraction of less than 3%, less than 2%, less than 1%, or less than 0.5%, when measured by HPLC.

In addition, the recycled bis(2-hydroxyethyl) terephthalate produced by the above process may have a yellow index (YID) of 3.0 or less as measured with a spectrophotometer. Specifically, the yellow index may be 2.5 or less, 2.0 or less, 1.5 or less, or 1.0 or less.

In addition, the recycled bis(2-hydroxyethyl) terephthalate produced by the above process may have a total content of inorganic substances of less than 5 ppm as measured by inductively coupled plasma atomic emission spectroscopy (ICP-AES). Specifically, the total content of inorganic substances may be less than 3 ppm, less than 1 ppm, or nearly 0 ppm.

### Mode for the Invention

Hereinafter, the present invention will be described in more detail with reference to embodiments. However, these examples are provided only for illustration purposes, and the present invention is not limited thereto.

### Example 1

A first reactor made of stainless steel (SUS) was charged with 1,000 g of a waste polyester resin pulverized to a particle size of 4 mm or less, 2,000 g of ethylene glycol, and 3.5 g of zinc acetate anhydride. The temperature inside the reactor was raised to 180°C, and depolymerization (first glycolysis reaction) was carried out for 2 hours.

The reactant (first reactant) thus obtained was transferred to a second reactor and cooled to 150°C. 2,000 g of ethylene glycol was further added thereto, and depolymerization (second glycolysis reaction) was carried out for 2 hours while the reactor temperature was maintained at 150°C.

The reactant (second reactant) thus obtained was cooled to 120°C through reduced pressure flash, and 16 g of a filter aid (Celite^{™} 545) was added thereto, followed by pressurized filtration to carry out solid-liquid separation.

The separated liquid reactant was passed through a column filled with an ion-exchange resin (BC107(H) of Bonlite) to remove ionic impurities to obtain a mixture (third reactant) containing bis(2-hydroxyethyl) terephthalate and ethylene glycol.

The mixture (third reactant) was transferred to a 10-liter distillation apparatus, and vacuum distillation was carried out at 130°C to recover unreacted ethylene glycol.

The reactant (fourth reactant) from which ethylene glycol had been removed was subjected to thin film evaporation at 220°C and 0.08 Torr in a thin film evaporator (VKL70-4S of VTA) to obtain 1,040 g of a product from which dimers or higher oligomers had been removed. Thereafter, for adsorption-crystallization, 1,040 g of the above product and 3,120 g of distilled water were charged to a 20-liter glass reactor, dissolved at a temperature of 70°C, and then 5.2 g of activated carbon was added thereto, followed by stirring for 30 minutes and filtration thereof. The filtrate was cooled to room temperature for the crystallization thereof, filtered, and dried in a vacuum oven. As a result, 990 g of a final product containing bis(2-hydroxyethyl) terephthalate was obtained.

### Example 2

980 g of a final product containing bis(2-hydroxyethyl) terephthalate was obtained through the same procedure as in Example 1, except that the first glycolysis reaction was carried out at 180°C for 1 hour.

### Example 3

985 g of a final product containing bis(2-hydroxyethyl) terephthalate was obtained through the same procedure as in Example 1, except that 1,000 g of a waste fiber was used as a raw material for waste polyester.

### Example 4

992 g of a final product containing bis(2-hydroxyethyl) terephthalate was obtained through the same procedure as in Example 1, except that 1,000 g of a waste banner was used as a raw material for waste polyester.

### Example 5

1,050 g of a final product containing bis(2-hydroxyethyl) terephthalate was obtained through the same procedure as in Example 1, except that no adsorption-crystallization step was carried out after the thin film evaporation.

### Comparative Example 1

A reactor made of stainless steel (SUS) was charged with 1,000 g of a waste polyester resin having a particle size of 4 mm or less, 4,000 g of ethylene glycol, and 3.5 g of zinc acetate anhydride. The temperature inside the reactor was raised to 196°C, and depolymerization (glycolysis reaction) was carried out for 4 hours. The reactant thus obtained was cooled to 30°C, and crystallization of bis(2-hydroxyethyl) terephthalate was carried out for 2 hours. The slurry of bis(2-hydroxyethyl) terephthalate and ethylene glycol thus obtained was subjected to solid-liquid separation in a centrifugal separator. Bis(2-hydroxyethyl) terephthalate obtained through centrifugation was washed twice with a sufficient amount of distilled water, and the residual solvent was removed in an oven to obtain about 1,010 g of a final product containing bis(2-hydroxyethyl) terephthalate.

### Comparative Example 2

About 1,000 g of a final product containing bis(2-hydroxyethyl) terephthalate was obtained through the same procedure as in Comparative Example 1, except that the glycolysis reaction was carried out at 210°C.

### Comparative Example 3

About 1,020 g of a final product containing bis(2-hydroxyethyl) terephthalate was obtained through the same procedure as in Example 1, except that the first glycolysis reaction was carried out at 196°C for 4 hours and that neither the second glycolysis reaction nor the adsorption-crystallization was carried out.

### Comparative Example 4

About 730 g of a final product containing bis(2-hydroxyethyl) terephthalate was obtained through the same procedure as in Example 1, except that the first glycolysis reaction was carried out at 150°C for 10 minutes and that the second glycolysis reaction was carried out at 190°C for 1 hour and 30 minutes.

In such an event, there were problems in the process as a significant amount of unreacted waste PET residue in the final reaction was filtered due to the low temperature of the first glycolysis reaction and the filtration took more than 10 hours, and the final yield was also reduced.

### Comparative Example 5

About 1,050 g of a final product containing bis(2-hydroxyethyl) terephthalate was obtained through the same procedure as in Example 1, except that the first glycolysis reaction was carried out at 210°C and that the second glycolysis reaction was carried out at 250°C.

### Comparative Example 6

About 995 g of a final product containing bis(2-hydroxyethyl) terephthalate was obtained through the same procedure as in Example 1, except that the unreacted glycol was recovered at 160°C.

### Test Example

The resultants (recycled bis(2-hydroxyethyl) terephthalate) of the Examples and Comparative Examples were tested as follows. The results are summarized in Table 1 below.

### (1) Composition - High performance liquid chromatography (HPLC)

About 0.01 g of a sample was diluted in about 20 ml of methanol and then measured by HPLC.
- Model: Waters e2695
- Column: C18 (4.6 × 250 mm), 5 µm
- UV detector: 242 nm
- Injection volume: 10 µl
- Eluent (gradient) A: H₂O+H₃PO₄, B: acetonitrile

### (2) Residual solvent - Gas chromatography (GC)

About 0.1 g of a sample was diluted in about 10 ml of CHCl₃, treated with a filter of 0.45 µm, and then measured by GC.
- Model: Agilent 7890B
- Column: DB-624 (30 m × 0.25 mm × 1.4 µm)
- Oven Temp.: 60°C (2 min) - 10°C/min - 200°C (0 min) - 20°C/min - 260°C (5 min)
- Injector temp.: 250°C
- Detector temp.: 250°C
- Flow: 1.5 ml/min (N2), split ratio: 1/50

### (3) Melting point - Differential scanning calorimetry (DSC)

Melting point (m.p.) was measured while heating from 30°C to 280°C at a rate of 10°C/minute using a differential scanning calorimeter (DSC, TA Instruments Q20).

### (4) Inorganic substances - Inductively coupled plasma atomic emission spectroscopy (ICP-AES)

0.3 g of a sample was treated with ultrasonic waves and diluted with ultrapure water. The content (ppm) of inorganic substances was measured using ICP-AES (Model 5100 of Agilent) (detection limit of 5 ppm).

### (5) Yield

The percentage by weight of bis(2-hydroxyethyl) terephthalate actually obtained in the Examples and Comparative Examples was calculated based on the maximum weight of bis(2-hydroxyethyl) terephthalate obtainable from 1,000 g of polyethylene terephthalate through a glycolysis reaction.

### (6) Yellow index (YID)

A sample was dissolved in dimethylformamide (DMF) at a concentration of 25% by weight at room temperature (23°C) to prepare a solution. Transmission data were obtained with Illuminant D65 using Color Flex EZ of Hunterlab at an observer's angle of 2° for the solution. The YID value was calculated using a color analyzer in the software.

**[Table 1]**

| | Info. | Ex. 1 | Ex. 2 | Ex. 3 | Ex. 4 | Ex. 5 | C. Ex. 1 | C. Ex. 2 | C. Ex. 3 | C. Ex. 4 | C. Ex. 5 | C. Ex. 6 |
|---|---|---|---|---|---|---|---|---|---|---|---|---|
| Yield | wt. % | 74.83 | 74.07 | 74.45 | 74.98 | 79.37 | 76.34 | 75.59 | 77.10 | 55.18 | 79.37 | 67.27 |
| HPLC (%) | BHET | 98.2 | 98.2 | 97.8 | 96.6 | 96.8 | 82.6 | 84.6 | 90.7 | 74.4 | 71.1 | 91.4 |
| | MHET | 1.1 | 1.1 | 1.1 | 1.7 | 1.9 | 1.0 | 2.2 | 1.7 | 1.9 | 2.7 | 1.8 |
| | DEG ester 1 | 0.4 | 0.4 | 0.6 | 0.6 | 0.8 | 3.7 | 5.4 | 2.9 | 1.1 | 9.6 | 2.2 |
| | DEG ester 2 | 0.0 | 0.0 | 0.2 | 0.1 | 0.1 | 0.4 | 0.3 | 0.3 | 0.2 | 1.1 | 0.9 |
| | Dimer | 0.2 | 0.2 | 0.2 | 0.8 | 0.2 | 10.4 | 6.4 | 3.7 | 18.6 | 13.2 | 1.3 |
| | Trimer | 0.0 | 0.0 | 0.0 | 0.0 | 0.0 | 0.9 | 0.7 | 0.4 | 2.9 | 2.1 | 2.1 |
| | Others | 0.1 | 0.1 | 0.1 | 0.2 | 0.2 | 1.1 | 0.5 | 0.5 | 1.0 | 0.2 | 0.2 |
| GC (wt. %) | Residual EG | 0.1 | 0.1 | 0.1 | 0.1 | 0.2 | 0.4 | 0.5 | 0.3 | 0.1 | 0.1 | 0.1 |
| DSC (°C) | m.p. | 112.2 | 111.9 | 112.1 | 111.5 | 112.5 | 110.5 | 108.5 | 111.1 | 108.2 | 102.3 | 110.7 |
| | Zn | N.D. | N.D. | N.D. | N.D. | N.D. | 80 | 50 | N.D. | N.D. | N.D. | N.D. |
| | Sb | N.D. | N.D. | N.D. | N.D. | N.D. | 5 | 8 | N.D. | N.D. | N.D. | N.D. |
| | Fe | N.D. | N.D. | N.D. | N.D. | N.D. | 11 | 7 | N.D. | N.D. | N.D. | N.D. |
| | P | N.D. | N.D. | N.D. | N.D. | N.D. | 22 | 23 | N.D. | N.D. | N.D. | N.D. |
| ICP-AES (ppm) | Mn | N.D. | N.D. | N.D. | N.D. | N.D. | N.D. | N.D. | N.D. | N.D. | N.D. | N.D. |
| | Mg | N.D. | N.D. | N.D. | N.D. | N.D. | N.D. | N.D. | N.D. | N.D. | N.D. | N.D. |
| | Al | N.D. | N.D. | N.D. | N.D. | N.D. | N.D. | N.D. | N.D. | N.D. | N.D. | N.D. |
| | Co | N.D. | N.D. | N.D. | N.D. | N.D. | N.D. | N.D. | N.D. | N.D. | N.D. | N.D. |
| | Na | N.D. | N.D. | N.D. | N.D. | N.D. | N.D. | N.D. | N.D. | N.D. | N.D. | N.D. |
| | K | N.D. | N.D. | N.D. | N.D. | N.D. | N.D. | N.D. | N.D. | N.D. | N.D. | N.D. |
| YID | - | 0.6 | 1.2 | 1.6 | 2.7 | 2.4 | 5.4 | 6.7 | 5.1 | 3.2 | 11.0 | 3.1 |
| * DEG ester 1: 2-hydroxyethyl[2-(2-hydroxyethoxy)ethyl] terephthalate, CAS No. 65133-69-9, | | | | | | | | | | | | |
| * DEG ester 2: bis[2-(2-hydroxyethoxy)ethyl]benzene-1,4-dicarboxylate, CAS No. 26850-76-0 | | | | | | | | | | | | |

As can be seen from the above table, in the products obtained in Examples 1 to 5, the ratio of BHET was high, no inorganic impurities were observed, the YID value was low, and the content of DEG-derived esters could be minimized. A polyester resin polymerized from the recycled BHET with high purity is excellent in thermal properties such as glass transition temperature, melting point, and crystallization temperature; thus, it can reproduce the structural regularity, molecular weight, and excellent thermal properties of a virgin polyester resin.

In contrast, the products obtained in Comparative Examples 1 and 5 had problems in that they contained a large amount of dimers or DEG-derived esters, had poor color quality due to a high yellowness index (YID), or had a significantly low yield.

## Claims

1. A process for preparing recycled bis(2-hydroxyethyl) terephthalate, which comprises:
(1) subjecting waste polyester to depolymerization by a first glycolysis reaction at a temperature of 180°C to 200°C to obtain a first reactant;
(2) subjecting the first reactant to depolymerization by a second glycolysis reaction at a temperature of 150°C to 170°C to obtain a second reactant;
(3) subjecting the second reactant to ion exchange through an ion-exchange resin to obtain a third reactant;
(4) removing an unreacted glycol from the third reactant through distillation at a temperature of 150°C or lower to obtain a fourth reactant; and
(5) subjecting the fourth reactant to distillation to obtain crude bis(2-hydroxyethyl) terephthalate.

2. The process for preparing recycled bis(2-hydroxyethyl) terephthalate of claim 1, wherein the waste polyester has a particulate or fibrous form with a particle diameter of 4 mm or less.

3. The process for preparing recycled bis(2-hydroxyethyl) terephthalate of claim 1, wherein the first glycolysis reaction in step (1) is carried out in the presence of a catalyst, and the catalyst comprises a metal acetate or an anhydride or a hydride thereof and is employed in an amount of 0.2 part by weight to 0.4 part by weight relative to 100 parts by weight of the waste polyester resin.

4. The process for preparing recycled bis(2-hydroxyethyl) terephthalate of claim 1, which further comprises, prior to the ion exchange in step (3), cooling the second reactant to 120°C or lower and filtering it upon the addition of a filter aid.

5. The process for preparing recycled bis(2-hydroxyethyl) terephthalate of claim 1, which further comprises, prior to the ion exchange in step (3), removing insoluble foreign substances from the second reactant through filtration.

6. The process for preparing recycled bis(2-hydroxyethyl) terephthalate of claim 1, wherein the ion-exchange resin in step (3) is used in an amount of 1 part by weight to 20 parts by weight relative to 100 parts by weight of the waste polyester and comprises at least one selected from the group consisting of a strongly acidic cation-exchange resin, a weakly acidic cation-exchange resin, and a chelate resin.

7. The process for preparing recycled bis(2-hydroxyethyl) terephthalate of claim 1, wherein the distillation for removing an unreacted glycol in step (4) is carried out at a temperature of 100°C to 130°C.

8. The process for preparing recycled bis(2-hydroxyethyl) terephthalate of claim 1, wherein the distillation to obtain crude bis(2-hydroxyethyl) terephthalate is carried out by thin film evaporation under a pressure of 0.05 Torr to 0.4 Torr.

9. The process for preparing recycled bis(2-hydroxyethyl) terephthalate of claim 1, which further comprises, after the distillation in step (5), adsorbing-crystallizing the crude bis(2-hydroxyethyl) terephthalate, wherein the adsorption-crystallization is carried out by adding an adsorbent using water as a solvent, filtering, and crystallization.

10. Recycled bis(2-hydroxyethyl) terephthalate, which is prepared by the process of claim 1, wherein the peak area fraction of bis(2-hydroxyethyl) terephthalate is 96% or more, and the peak area fraction of diethylene glycol (DEG) ester compounds is less than 2% in total, when measured by high-performance liquid chromatography (HPLC).

11. The recycled bis(2-hydroxyethyl) terephthalate of claim 10, wherein the recycled bis(2-hydroxyethyl) terephthalate has a peak area fraction of oligomers of 3% or less in total when measured by high-performance liquid chromatography.

12. The recycled bis(2-hydroxyethyl) terephthalate of claim 10, wherein the recycled bis(2-hydroxyethyl) terephthalate has a yellow index (YID) of 3.0 or less as measured with a spectrophotometer.

13. The recycled bis(2-hydroxyethyl) terephthalate of claim 10, wherein the recycled bis(2-hydroxyethyl) terephthalate has a total content of inorganic substances of less than 5 ppm as measured by inductively coupled plasma atomic emission spectroscopy (ICP-AES).
